# EUROPEAN PATENT SPECIFICATION

(11) **EP 1 833 446 B1**
(45) Date of publication and mention of the grant of the patent: **29.08.2012**
(21) Application number: 04809072.4
(22) Date of filing: 16.12.2004
(51) Int. Cl.: A61F 13/56, A61F 13/84, A61F 13/64

(54) **ABSORBENT ARTICLE COMPRISING A BELT**
EINEN GURT ENTHALTENDER SAUGFÄHIGER ARTIKEL
ARTICLE ABSORBANT COMPRENANT UNE CEINTURE

(43) Date of publication of application: 19.09.2007
(73) Proprietor: SCA Hygiene Products AB, 405 03 Göteborg (SE)
(72) Inventor: MELANDER, Magnus, S-412 62 Göteborg (SE)
(74) Representative: Börlin, Maria
(86) International application number: PCT/SE2004/001899
(87) International publication number: WO 2006/065177

(56) References cited:
- US-A1- 2002 091 369
- US-B1- 6 322 552
- US-B2- 6 626 882

## Description

### TECHNICAL FIELD

The invention relates to an absorbent article for disposable use such as a diaper or an incontinence guard exhibiting an absorbent part. The absorbent part exhibits a longitudinal direction and a transverse direction as well as a front end part, a rear end part and an interjacent crotch part. The article further exhibits a belt that is attached to the rear end part, or alternatively the front end part, of the absorbent part. The belt extends in the transverse direction outside at least one of the longitudinal edges of the absorbent part.

The belt is intended to be joined together around a wearer's waist, in conjunction with which the belt exhibits at least one fixing device intended to attach the aforementioned belt around the wearer's waist. The rear end part, or alternatively the front end part, exhibits fixing devices intended to be attached to the side of the belt that is intended to face away from the wearer during use, as a consequence of which the article adopts a form resembling panties, in which the belt forms a part of the waist part of the article.

### BACKGROUND

Absorbent articles such as disposable diapers for infants or incontinence diapers for adult users are often indented to be secured around the wearer's waist. A commonly encountered type of absorbent article accordingly comprises securing flaps arranged on the rear end part of the article. The securing flaps are intended to be attached to the front end part of the article when the article has been put on a wearer. Special receiving surfaces specially adapted for receiving the securing flaps are commonly encountered.

It is common, for example, for the securing flaps to incorporate Velcro@ elements of the male type and for the receiving surfaces to incorporate Velcro@ elements of the female type.

Another commonly encountered type of absorbent article consists of so-called belt diapers, which are particularly commonly encountered for adult incontinent users.
Belt diapers are characterized in that they comprise a transverse belt attached to either the front or the rear transverse edge of the absorbent part of the diaper, in conjunction with which the opposite transverse edge of the absorbent part is free.
When putting on a belt diaper of this kind, the belt is fixed, in a first stage, around the wearer's waist. The free transverse edge of the absorbent part of the diaper is then passed between the wearer's legs and is attached to the side of the belt facing away from the wearer.
Special securing devices are arranged on the free transverse edge of the absorbent part of the diaper to enable the free transverse edge of the absorbent part to be secured to the belt. The securing devices are intended to be secured to the side of the belt that faces away from the wearer. Special receiving surfaces on the belt designed to interact with the securing devices are commonly encountered. It is also a commonly occurring situation for the entire belt to be made from a material that is capable of interacting with the securing devices on the absorbent part, in conjunction with which no special receiving surfaces need to be arranged on the belt.
Belt diapers are described in a number of different patent documents. Belt diapers are described, for example, in international patent application WO 2002049568 and in Swedish patent No. 514 370.

In connection with the care of incontinent wearers in an institutional environment, it is a common occurrence for a diaper change to be performed during the night. In order to avoid waking up other patients who are sleeping in the same room, it is customary for changes that are made during the night to be performed with only night lighting lit.

A problem encountered in conjunction with changing under low lighting conditions/in semi-darkness is that of locating the belt of the diaper against the bedclothes.

Another problem, which is encountered not only in conjunction with the use of belt diapers, but also in conjunction with the use of other types of diaper, is that the nursing staff may fail to discover that a soiled diaper is lying among the bedclothes that will be sent to the laundry. It is customary for all bedclothes to be gathered together in a large bundle that is placed in a laundry bag, in which case the overlooked diaper finds its way to the laundry, where the diaper is unpleasant for the laundry workers and can cause operating interruptions such as blocked drains, for example.

The need accordingly exists for an improved belt diaper where the care provider can more easily locate the belt, including when the diaper change is performed under conditions of limited lighting.

The need also exists for an improved belt diaper that can be found and removed from the bedclothes more easily when these are replaced.

### DESCRIPTION OF THE INVENTION

An absorbent article according to claim 1 essentially overcomes the problems referred to initially that have been associated with previous articles of this kind.

The fact that the belt is easy to distinguish from light-colored bed textiles, for example, facilitates changing the belt diaper when the wearer is lying in bed at the time of the change. There is also a substantially smaller risk of the belt diaper finding its way along with the bed textiles into a laundry bag, for example, when the bedclothes are changed.
Because most types of bed textiles consist of light-colored or white textiles, it is especially important for there to be a good ability to be distinguished from essentially light-colored or white bedclothes.

A good ability to be distinguished can be achieved without the entire belt needing to exhibit the aforementioned ability to be distinguished. It may be sufficient for a part of the belt to exhibit the ability to be distinguished.

According to an alternative embodiment, the whole of one side of the belt exhibits distinguishing means.

In accordance with one embodiment, both sides of the belt exhibit distinguishing means.

The ability to be distinguished on both sides of the belt further increases the likelihood that the belt will be discovered quickly, including in a partially darkened room.

One embodiment is characterized in that the distinguishing means of the first side and the distinguishing means of the second side differ from one another.

In one embodiment, the distinguishing means comprises a patterned area, in conjunction with which the pattern comprises a color that deviates from that of the belt.

One embodiment is characterized in that the distinguishing means comprises an embossed area that can be detected with the help of the sense of touch.

The invention is characterized in one embodiment in that the belt comprises a first belt half and a second belt half. The first belt half extends in the transverse direction outside one longitudinal edge of the absorbent part, and the second belt half extends in the transverse direction outside the opposite longitudinal edge of the absorbent part.
A belt diaper that exhibits belt halves in the transverse direction in both directions is relatively easier to secure on a wearer, in conjunction with which the two belt halves are attached to one another on the wearer's abdomen.

In one embodiment, both belt halves exhibit distinguishing means.

One embodiment is characterized in that the belt consists of two separate belt halves, in conjunction with which the respective belt half is attached to the absorbent part of the belt diaper.

In another embodiment, the belt consists of a single piece of material, in conjunction with which the two belt halves constitute opposite end areas of the piece of material. The belt, which consists of a single piece of material, is attached to the absorbent part of the belt diaper along the whole of the front or rear transverse edge, as a consequence of which a very durable belt is obtained.

### BRIEF DESCRIPTION OF THE FIGURE

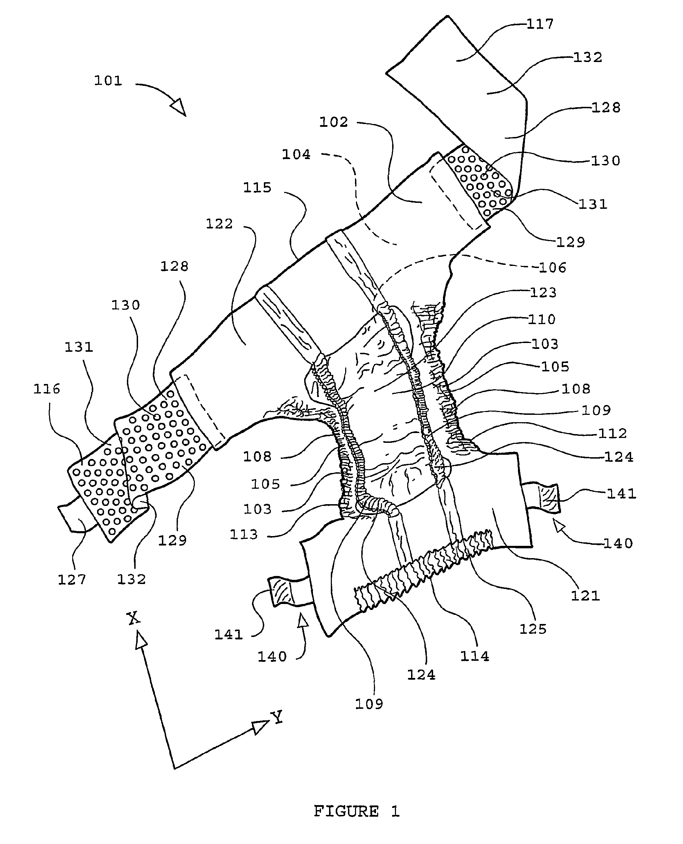

- Figure 1: shows a belt diaper in accordance with a preferred embodiment of the invention from the side that is intended to face towards the wearer during use.

### DESCRIPTION OF THE ILLUSTRATIVE EMBODIMENTS

The invention relates to a so-called belt diaper, in conjunction with which the diaper comprises a belt intended to surround a wearer's waist when it is being worn.

Figure 1 shows important components of a belt diaper 101 in accordance with the invention, in conjunction with which the belt diaper is shown from the side that is intended to face towards the wearer when it is being worn.

The belt diaper 101 exhibits a longitudinal direction X and a transverse direction Y and comprises an absorbent part 110 and an elastic belt 128.

The elastic belt 128 of the belt diaper 101 is intended to surround a wearer's waist, in conjunction with which the belt 128 exhibits a first belt half 116 and a second belt half 117. The belt halves 116,117 extend in the transverse direction Y of the belt diaper.
The belt halves 116,117 are attached to the absorbent part 110 on the rear end part 122, in conjunction with which the first belt half 116 is joined to the longitudinal edge 113, and the second belt half 117 is joined to the opposing longitudinal edge 112.
The first belt half 116 comprises a fixing device 127 for fixing the belt 128 around a wearer's waist, in conjunction with which the fixing device comprises a Velcro@ surface which interacts with the second belt half 117 during use.

The designation belt half does not denote that the two belt halves must be of identical length. In alternative embodiments, one belt half can be longer than the other. It is important, however, for the combined length of both parts of the belt to reach around a wearer's waist.

In an alternative embodiment, the two belt halves 116,117 of the belt diaper 101 can consist of only a single piece of material, in conjunction with which the piece of material extends in the transverse direction over the whole of the rear end part 122 and past the respective longitudinal edge 112,113.

In another alternative embodiment, it is possible to envisage that the belt diaper 101 exhibits only a single belt part, in conjunction with which the belt part extends in the transverse direction past only one of the longitudinal edges 112,113 of the belt diaper 101. The single belt part in such an embodiment exhibits a length which permits the belt part to reach around the waist of a wearer.

The belt 128 can be inelastic or partially elastic in other alternative embodiments. The designation partially elastic belt 128 denotes that certain parts of the length of the belt 128 exhibit elastic properties, while certain other parts of the length of the belt exhibit non-elastic properties.

The belt diaper 101 also exhibits two front fixing devices 140 arranged on the longitudinal edges 112,113 of the absorbent part 110 of the belt diaper 101 on the front end part 121 of the belt diaper 101. The front fixing devices 140 are intended to be fixed to the belt 128 in conjunction with putting the belt diaper 101 on a wearer.
The front fixing devices 140 comprise Velcro@ elements 141 intended to interact with the side of the belt 128 which is intended to face away from the wearer during use.

In alternative embodiments, the front fixing devices 140 can comprise adhesive elements intended to be fixed to the side of the belt 128 facing away from the wearer. The belt 128 in this case must comprise surfaces intended for interaction with the adhesive elements, or must be made from a suitable material to interact with the adhesive elements. For example, the belt 128 can comprise surfaces of polyethylene film that are suitable surfaces for interaction with the adhesive elements on the fixing devices 140.

In other alternative embodiments, the front fixing devices 140 can be arranged directly on the liquid-permeable covering layer 102 of the belt diaper 101, in conjunction with which they are arranged at the front corners of the absorbent part 110, that is to say where the front transverse edge 114 meets the respective longitudinal edge 112,113.

It is also possible to envisage a single front fixing device 140 arranged on the liquid-permeable covering layer 102 of the belt diaper 101, in conjunction with which the fixing device 140 is long and narrow and extends along essentially the whole of the front transverse edge 114.

When the belt diaper 101 is to be put on a wearer, the belt 128 is secured around the wearer's waist as a first stage.
The absorbent part 110 of the belt diaper 101 comprising, among other things, the front transverse edge 114 and the absorption body 106, is then passed between the wearer's legs, after which the two front fixing devices 140 are secured to the belt 128 on the side facing away from the wearer.

The absorbent part 110 of the belt diaper 101 is essentially in the form of an hourglass and as such exhibits longitudinal edges 112, 113, a front transverse edge 114 and a rear transverse edge 115. The diaper 101 also exhibits a front end part 121, a rear end part 122 and a narrower crotch part 123 situated between the end parts 121, 122. The crotch part 123 is intended to be situated in the narrowest area between the wearer's thighs when it is being worn.
When wearing the diaper 101, the front part of the crotch part 123 and the front end part 121 function principally as a receiving area for urine, while the rear part of the crotch part 123 and the rear end part 122 function principally as a receiving area for feces.

The diaper 101 comprises a liquid-permeable covering layer 102 arranged over the surface of the diaper 101 that is intended to face towards the wearer when it is being worn, a backing layer 104 arranged over the surface of the diaper that is intended to face away from the wearer when it is being worn, an absorption body 106 enclosed between the liquid-permeable covering layer 102 and the backing layer 104, and side flaps 103 arranged outside the absorption body 106.

The liquid-permeable covering layer 102 of the diaper 101 extends outside the absorption body 106 around the periphery of the entire absorption body 106. The liquid-permeable covering layer 102 can consist of any material that is suitable for the purpose. Examples of commonly encountered liquid-permeable covering materials are non-woven textile materials, known as nonwoven materials, perforated plastic films, meshes made of plastic or textile, and liquid-permeable foam layers. Liquid-permeable covering materials which consist of continuous thin fibers that extend predominantly in the longitudinal or transverse direction of the article are also encountered. Laminates consisting of two or more of the above-mentioned possible covering materials are also commonly encountered, as are coverings consisting of different materials in different parts of the surface.
A situation commonly encountered today is for the liquid-permeable covering layer 102 to consist of a fully or partially elastic material in order to provide the diaper 101 with a better fit when it is being worn.

Diapers 101 containing absorption bodies 106 which exhibit especially high strength and resistance to wear may even function without the need to provide any extra liquid-permeable covering layer on the side of the diaper 101 that faces towards the wearer when it is being worn.

The backing layer 104 also extends beyond the absorption body 106 around the periphery of the entire absorption body 106. Backing layers 104 that are normally present on diapers 101 are usually liquid-impermeable, although other types of backing layer are also encountered. The backing layer 104 can consist of a range of different materials. It is most common for the backing layer 104 to consist of a thin and preferably liquid-tight plastic film, although it is also possible to use other types of liquid-tight material such as nonwoven materials that have been made liquid-tight, for example by means of plastic coating, liquid-tight foam layers, liquid-tight adhesive or similar.

The backing layer 104 can also consist of a liquid-tight, vapor-permeable material known as a breathable material.

Also encountered are laminates containing at least one preferably liquid-tight layer arranged against the absorption body 106. These laminates usually consist of a liquid-tight material functioning as a moisture barrier and a more textile-like material arranged on the side of the diaper 101 that faces away from the wearer when it is being worn, as a consequence of which the outside of the diaper 101 more closely resembles an item of clothing when it is being worn. The textile-like layer of the laminate usually consists of a nonwoven layer, although other textile or textile-like materials are also encountered.

The liquid-permeable covering layer 102 and the backing layer 104 are attached to one another outside the absorption body 106 along the entire periphery of the absorption body 106.

The liquid-permeable covering layer 102 and the backing layer 104 may be attached to one another by a number of different means. Examples of means of attachment include gluing, thermal fusion, ultrasonic welding or the like.

Elastic devices 105 are arranged outside the absorption body 106 in those parts of the side flaps 103 of the belt diaper 101 which run essentially in the longitudinal direction of the belt diaper 101. The elastic devices 105 function as leg elastic and have the task of preventing liquid and feces from leaking out past the longitudinal edges 112, 113 of the diaper 101, and in this way, together with surrounding layers, they form outer moisture barriers 108 of the belt diaper 101. The elastic devices 105 consist of one or more elastic threads that have been applied in their stretched state between the liquid-permeable covering layer 102 and the backing layer 104, at least in the crotch part 123 of the diaper 101. The elastic devices 105 are attached to the backing layer 104 and the covering layer 102 by gluing, ultrasonic welding or the like.

In alternative embodiments, the elastic devices can be arranged on the side of the side flaps 103 that is intended to face towards the wearer during use, or on the opposite side of the side flaps, and as such they are naturally only attached to the covering layer 102 and the backing layer 104 respectively.

The elastic devices can, in alternative embodiments, consist of elastic tape material, for example made of a foam material.

The hourglass-shaped absorption body 106 can be constructed from one or more layers of cellulose fluff pulp. The cellulose fluff pulp can be mixed for this purpose with fibers or particles of a high-absorbency polymer material, known as superabsorbents, of the kind which, in conjunction with absorption, chemically bonds large quantities of liquid to form a liquid-containing gel. The absorption body 106 can also contain high-absorbency polymer material arranged in a layer inside the absorption body or in conjunction with the surface or surfaces of the absorption body. Additional components to improve the characteristics of the absorption body 106 can also be present in the absorption body 106. Examples of such components include binding fibers, different types of liquid-distributing layers or fibers, form-stabilizing components, reinforcing fibers or the like. The absorption body 106 can naturally also consist of other types of absorption material, such as absorbent nonwoven material, absorbent foam, textile materials, peat or mixtures of different kinds of absorption material.

Special layers with the ability rapidly to receive quite large quantities of liquid and to retain this liquid temporarily, in order subsequently to release the temporarily stored liquid to other parts of the absorption body 106, can also be incorporated in diapers of the prescribed kind. Such receiving layers are normally arranged for this purpose between the liquid-permeable covering layer 102 of the diaper 101 and the absorption body 106. No receiving layer is shown in Figure 1.

In order further to prevent liquid or feces from leaking out via the side edges 112, 113 of the diaper 101, the diaper 101 is provided with inner side leakage barriers 109 on the side that is intended to face towards the wearer when it is being worn. The inner side leakage barriers 109 are arranged on either side of and adjacent to the longitudinal edges 110 of the absorption body 106, and they extend essentially in the longitudinal direction of the diaper 101. The respective inner side leakage barrier 109 is executed from a separate material strip, which exhibits two essentially parallel longitudinal edges. The material strip is double-folded, in conjunction with which the parallel longitudinal edges are arranged adjacent to one another. The edges of the material strip are attached to the covering layer 102 and constitute the attached edge of the respective inner side leakage barrier. The folded edge of the material strip constitutes the free edge of the inner side leakage barrier 109.

The inner side leakage barriers 109 are folded down and attached to the covering layer 102 on the front end part 121 and the rear end part 122 of the diaper 101.

The inner side leakage barriers 109 comprise elastic elements 124 attached to the inner side leakage barriers 109 in a pre-tensioned state. The elastic elements 124 are conveniently arranged adjacent to the free edges of the inner side leakage barriers 109. When the pre-tensioned elastic elements 124 are released, they contract together with the free edges of the inner side leakage barriers 109, thereby causing the inner side leakage barriers 109 to be brought into a raised configuration remote from the liquid-permeable covering layer 102, at least, in the crotch part 123 of the diaper 101, where the side leakage barriers 109 are not folded down and attached to the covering layer 102.

At least one or other of the rear or front end parts of the absorbent part 110 can be provided with so-called waist elastic 125, which consists of elastic devices arranged along the front transverse edge 114 or the rear transverse edge 115 of the belt diaper 101 in order to provide the diaper 101 with a soft and pliable closure around the wearer's waist. In the illustrative embodiment described here, only the front end part 121 of the diaper 101 is provided with waist elastic 125. The waist elastic 125 consists of a thin strip of elastic foam material, which is attached by means of adhesive between the backing layer 104 and the liquid-permeable surface layer 102. The waist elastic 125 is applied in its stretched state between the layers 102, 104 in order to bring about a holding force which stretches the diaper 101 around the wearer's waist. When the front fixing devices 140 are secured to the belt 128, it is appropriate for the waist elastic 125 to be stretched out in the transverse direction of the belt diaper, in conjunction with which the front end part 121 adopts a smooth configuration over the wearer's abdomen when it is being worn.

The belt diaper 101 is characterized primarily in that the belt 128 exhibits distinguishing means 129, in conjunction with which the belt 128 is considerably easier to detect against, for example, essentially light-colored bedclothes or against other parts of the belt diaper 101. The distinguishing means 129 in the embodiment described here consists of an embossed pattern 130 made up of colored points, although in alternative embodiments it can consist of any alternative pattern at all, that is distinguishable from the commonly encountered surroundings for belt diapers 101. Normally encountered surroundings include, for example, light-colored or white bedclothes. The fact that the pattern is embossed means that the belt can be distinguished from the surroundings including by the sense of touch on the wearer.

In the embodiment described here, the sides 131 that are intended to face towards the wearer during use exhibit a pattern 130, as a consequence of which the sides 131 are patterned on both belt halves 116,117. Opposing sides 132 of the belt halves 116,117 exhibit no pattern, although they can naturally also be patterned in alternative embodiments.

A large number of alternative embodiments are conceivable with regard to the sides 131,132, and the size of the area on these sides 131,132, that can be patterned or unpatterned.

As a minimum variant of a distinguishing means 129, it is possible to envisage an embodiment in which one side 131,132 of one belt half 116,117 exhibits a pattern 130 over a substantial part of its total area, where the expression substantial part denotes that more than 50% of the side 131,132 is patterned.

In the alternative embodiment described above, which exhibits only a substantially longer belt part which extends in the transverse direction past only one of the longitudinal edges 112,113 of the belt diaper 101, it is possible to envisage, as a minimum variant, that more than 25% of one side of the belt constitutes a substantial part of the belt.

In an embodiment which may be regarded as a form of maximum variant of the distinguishing means 129, it is possible to envisage that both sides 131,132 of both belt halves 116,117 comprise patterns 130. Moreover, the patterns 130 cover the whole of the sides 131,132 on the belt halves 116,117 in the envisaged maximum variant. When both sides of a belt half comprise patterned areas, the patterns on both sides should be distinguishable from one another.

As alternative intermediate variants of embodiments, varying numbers of sides 131,132 can exhibit patterns 130, in conjunction with which the sides 131,132 can be fully patterned or partially patterned.

In further alternative embodiments, it is possible to envisage that the patterned surfaces are replaced by colored surfaces, which can be of a single color or can incorporate a color pattern. It is also possible to use fluorescent colors in order to increase the ability to be distinguished in the dark. It is also possible to combine such colored areas with embossed parts.

Belt diapers 101 in accordance with the embodiment described here are especially suitable for wearers who are able to stand up when changing of the diaper 101 is performed. The normal procedure for such a change situation involves, as a first stage, securing the belt 128 around the wearer's waist so that the absorbent part 110 hangs down centrally to the rear of the wearer, as a consequence of which the absorbent part 110 hangs down freely towards the floor. In subsequent application stages, the absorbent part 110 of the belt diaper 101 is introduced between the wearer's legs and is secured to the part of the belt 128 that is situated to the front of the wearer by means of the front fixing devices 140.

In the case of users who are confined to bed, it is not as easy to put on a belt diaper 101, especially if the diaper needs to be changed in semi-darkness with only night lighting lit, although the many other advantages of the belt diaper 101 mean that it is nevertheless a commonly occurring situation for people who are confined to bed to wear belt diapers 101.
In conjunction with changing, for example, the belt 128 must be introduced under the abdomen of the person who is confined to bed and secured around the waist, in conjunction with which the belt 128 must be capable of being located without excessive difficulty in semi-darkness among usually white bedclothes.
The belt 128 must then be applied around the wearer's waist in a correct fashion without being twisted or positioned incorrectly in some other way. In these two application stages in particular, a major advantage is associated with the ability of the belt 128 to be distinguished from the bedclothes.

There is a further advantage if both sides 131,132 of the belt 128 can be distinguished from one another.

An additional problem that is solved for a belt diaper 101 in accordance with the present invention is the not entirely unfamiliar problem that diapers find their way unintentionally into the laundry in conjunction with changing bedclothes. The problem applies to diapers of all kinds, but it is solved in an effective fashion for belt diapers 101 in accordance with the present invention.

Changing the bedclothes in an institutional care environment is often performed as a consequence of the diaper having leaked, in conjunction with which the bedclothes are saturated and are often additionally contaminated with feces. In such situations, the used diaper is usually removed from the wearer, after which the latter is removed from the bed since the bedclothes require to be changed. In order to minimize the inconvenience of having to deal with the malodorous and soiled bedclothes, it is customary for the nursing staff rapidly to gather together all the bedclothes in a large bundle that is placed immediately in a laundry bag or the like. If the diaper is left behind among the bedclothes in conjunction with this, it is very easy for the diaper to find its way into the bundle of bedclothes, especially if the whole diaper lacks the ability to be distinguished from the bedclothes. Because the belt 128 on the belt diaper 101 in accordance with the invention comprises distinguishing means 129 and exhibits the ability to be distinguished from the bedclothes, the likelihood of the belt diaper 101 being discovered and removed from the bedclothes before these are placed in the laundry bag is increased.

The invention also extends to all conceivable combinations of the described illustrative embodiments.

Furthermore, the invention is not restricted to the above-mentioned illustrative embodiments, but is naturally applicable to other embodiments within the scope of the following patent claims.

## Claims

1. An absorbent article (101) for disposable use such as a diaper or an incontinence guard exhibiting an absorbent part (110), in conjunction with which the absorbent part (110) exhibits a longitudinal direction (X) and a transverse direction (Y) as well as a front end part (121), a rear end part (122) and an interjacent crotch part (123), in conjunction with which the article (101) exhibits a belt (128) attached to the rear end part (122) or alternatively the front end part (121) of the absorbent part (110), in conjunction with which the belt (128) extends in the transverse direction (Y) outside at least one of the longitudinal edges (112,113) of the absorbent part (110), at least one side (131,132) of the belt (128) exhibits distinguishing means (129) comprising a colored surface, **CHARACTERIZED IN THAT** at least 50% of one side (131,132) of the belt (128) exhibits distinguishing means (129).

2. An absorbent article (101), as claimed in claims 1
**CHARACTERIZED IN THAT**
the whole of one side (131,132) of the belt (128) exhibits distinguishing means (129).

3. An absorbent article (101), as claimed in one or other of the foregoing claims,
**CHARACTERIZED IN THAT**
both sides (131,132) of the belt (128) exhibit distinguishing means (129).

4. An absorbent article (101), as claimed in claim 3,
**CHARACTERIZED IN THAT**
the distinguishing means of the first side (131) and the distinguishing means of the second side (132) differ from one another.

5. An absorbent article (101), as claimed in one or other of claims 1 - 4,
**CHARACTERIZED IN THAT**
the distinguishing means (129) comprises a patterned area, in conjunction with which the pattern (130) comprises a color that deviates from that of the belt (128).

6. An absorbent article (101), as claimed in one or other of the foregoing claims,
**CHARACTERIZED IN THAT**
the distinguishing means (129) comprises an embossed area that can be detected with the help of the sense of touch.

7. An absorbent article (101), as claimed in one or other of the foregoing claims,
**CHARACTERIZED IN THAT**
the belt (128) comprises a first belt half (116) and a second belt half (117), in conjunction with which the first belt half (116) extends in the transverse direction (Y) outside one longitudinal edge (113) of the absorbent part (110) and the second belt half (117) extends in the transverse direction (Y) outside the opposite longitudinal edge (112) of the absorbent part (110).

8. An absorbent article (101), as claimed in claim 7,
**CHARACTERIZED IN THAT**
both belt halves (116,117) exhibit distinguishing means (129).

9. An absorbent article (101), as claimed in claims 7 or 8,
**CHARACTERIZED IN THAT**
the belt (128) consists of two separate belt halves (116,117), in conjunction with which the respective belt half (116,117) is attached to the absorbent part (110) of the belt diaper (101).

10. An absorbent article (101), as claimed in one or other of claims 7 or 8,
**CHARACTERIZED IN THAT**
the belt (128) consists of a piece of material, in conjunction with which the two belt halves (116,117) constitute opposite end areas of the piece of material.

## Patentansprüche

1. Saugfähiger Artikel (101) zur Einweganwendung, wie eine Windel oder ein Inkontinenzschutz, aufweisend einen absorbierenden Teil (110), wobei der absorbierende Teil (110) eine Längsrichtung (X) und eine Querrichtung (Y) sowie einen Vorderendteil (121), einen Hinterendteil (122) und einen dazwischenliegenden Schrittteil (123) aufweist, wobei der Artikel (101) einen Gurt (128) aufweist, der mit dem Hinterendteil (122) oder alternativ dem Vorderendteil (121) des absorbierenden Teils (110) verbunden ist, wobei der Gurt (128) sich in der Querrichtung (Y) außerhalb mindestens einer der länglichen Kanten (112, 113) des absorbierenden Teils (110) erstreckt, mindestens eine Seite (131, 132) des Gurts (128) ein eine gefärbte Oberfläche umfassendes Kennmittel (129) aufweist, **dadurch gekennzeichnet, dass** mindestens 50 % einer Seite (131, 132) des Gurts (128) Kennmittel (129) aufweist.

2. Saugfähiger Artikel (101) nach Anspruch 1, **dadurch gekennzeichnet, dass** die gesamte eine Seite (131,132) des Gurts (128) Kennmittel (129) aufweist.

3. Saugfähiger Artikel (101) nach irgendeinem der vorhergehenden Ansprüche, **dadurch gekennzeichnet, dass** beide Seiten (131, 132) des Gurts (128) Kennmittel (129) aufweisen.

4. Saugfähiger Artikel (101) nach Anspruch 3, **dadurch gekennzeichnet, dass** das Kennmittel der ersten Seite (131) und das Kennmittel der zweiten Seite (132) sich von einander unterscheiden.

5. Saugfähiger Artikel (101) nach irgendeinem der Ansprüche 1-4, **dadurch gekennzeichnet, dass** das Kennmittel (129) einen gemusterten Bereich umfasst, wobei das Muster (130) eine Farbe aufweist, die von der des Gurts (128) abweicht.

6. Saugfähiger Artikel (101) nach irgendeinem der vorhergehenden Ansprüche, **dadurch gekennzeichnet, dass** das Kennmittel (129) einen geprägten Bereich umfasst, der durch Tasten erkannt werden kann.

7. Saugfähiger Artikel (101) nach irgendeinem der vorhergehenden Ansprüche, **dadurch gekennzeichnet, dass** der Gurt (128) eine erste Gurthälfte (116) und eine zweite Gurthälfte (117) umfasst, wobei sich die erste Gurthälfte (116) in der Querrichtung (Y) außerhalb einer länglichen Kante (113) des absorbierenden Teils (110) und sich die zweite Gurthälfte (117) in der Querrichtung (Y) außerhalb der gegenüberliegenden länglichen Kante (112) des absorbierenden Teils (110) erstreckt.

8. Saugfähiger Artikel (101) nach Anspruch 7, **dadurch gekennzeichnet, dass** die beiden Gurthälften (116, 117) Kennmittel (129) aufweisen.

9. Saugfähiger Artikel (101) nach Anspruch 7 oder 8,
**dadurch gekennzeichnet, dass** der Gurt (128) aus zwei getrennten Gurthälften (116, 117) besteht, wobei die jeweilige Gurthälfte (116, 117) mit dem absorbierenden Teil (110) der Gurtwindel (101) verbunden ist.

10. Saugfähiger Artikel (101) nach irgendeinem der Ansprüche 7 oder 8,
**dadurch gekennzeichnet, dass** der Gurt (128) aus einem Materialstück besteht, wobei die zwei Gurthälften (116, 117) gegenüberliegende Endbereiche des Materialstücks ausmachen.

## Revendications

1. Article absorbant (101) pour utilisation jetable, tel qu'une couche-culotte ou une protection contre l'incontinence, présentant une partie absorbante (110), la partie absorbante (110) présentant une direction longitudinale (X) et une direction transversale (Y) ainsi qu'une partie d'extrémité avant (121), une partie d'extrémité arrière (122) et une partie d'entrejambe intercalée (123), l'article (101) présentant une ceinture (128) fixée à la partie d'extrémité arrière (122) ou alternativement la partie d'extrémité avant (121) de la partie absorbante (110), la ceinture (128) s'étendant dans la direction transversale (Y) à l'extérieur d'au moins l'un des bords longitudinaux (112, 113) de la partie absorbante (110), au moins un côté (131, 132) de la ceinture (128) présentant des moyens de distinction (129) avec une surface colorée, **caractérisé en ce qu'**au moins 50% d'un côté (131, 132) de la ceinture (128) présente des moyens de distinction (129).

2. Article absorbant (101) selon la revendication 1, **caractérisé en ce que** l'ensemble d'un côté (131, 132) de la ceinture (128) présente des moyens de distinction (129).

3. Article absorbant (101) selon l'une quelconque des revendications précédentes, **caractérisé en ce que** les deux côtés (131, 132) de la ceinture (128) présentent des moyens de distinction (129).

4. Article absorbant (101) selon la revendication 3, **caractérisé en ce que** les moyens de distinction du premier côté (131) et les moyens de distinction du deuxième côté (132) diffèrent les uns des autres.

5. Article absorbant (101) selon l'une quelconque des revendications 1 à 4, **caractérisé en ce que** le moyen de distinction (129) comprend une zone à dessin, le motif (130) comprenant une couleur qui diffère de celle de la ceinture (128).

6. Article absorbant (101) selon l'une quelconque des revendications précédentes, **caractérisé en ce que** le moyen de distinction (129) comprend une zone gaufrée qui peut être détectée à l'aide du sens du toucher.

7. Article absorbant (101) selon l'une quelconque des revendications précédentes, **caractérisé en ce que** la ceinture (128) comprend une première moitié de ceinture (116) et une deuxième moitié de ceinture (117), la première moitié de ceinture (116) s'étendant dans la direction transversale (Y) à l'extérieur d'un bord longitudinal (113) de la partie absorbante (110), et le deuxième moitié de ceinture (117) s'étendant dans la direction transversale (Y) à l'extérieur du bord longitudinal opposé (112) de la partie absorbante (110).

8. Article absorbant (101) selon la revendication 7, **caractérisé en ce que** les deux moitiés de ceinture (116, 117) présentent des moyens de distinction (129).

9. Article absorbant (101) selon les revendications 7 ou 8, **caractérisé en ce que** la ceinture (128) est composée de deux moitiés de ceinture distinctes (116, 117), la moitié de ceinture respective (116, 117) étant attachée à la partie absorbante (110) de la couche-culotte de ceinture (101).

10. Article absorbant (101) selon l'une quelconque des revendications 7 ou 8, **caractérisé en ce que** la ceinture (128) est constitué d'une pièce de matériau, les deux moitiés de ceinture (116, 117) étant composées des zones d'extrémité opposées de la pièce de matériau.
